# EUROPEAN PATENT APPLICATION

(11) **EP 4 397 267 A1**
(43) Date of publication of application: **10.07.2024**
(21) Application number: 23150750.0
(22) Date of filing: 09.01.2023
(51) Int. Cl.: A61B 34/00, A61B 46/10, A61N 1/36, A61B 34/30

(54) **CONVEYED MOTION APPARATUS**

(30) Priority: 06.01.2023 EP 23150619
(71) Applicant: CAScination AG, 3008 Bern (CH)
(72) Inventor: MATULIC, Marco, 3008 Bern (CH)
(74) Representative: Schulz Junghans Patentanwälte PartGmbB

(57) **Abstract**

A system for conveying motion from a non-sterilizable component to a sterilizable component is provided. Motion is conveyed through the coupling of a ferromagnetic source in the non-sterilizable component to a ferromagnetic sink attached to the sterilizable component. The ferromagnetic sink may be configured as an adapter that can be further coupled to tools for advancing or manipulating objects in a sterile field. A system for surgical implantation medical devices comprising an insertion tool is also provided. The insertion tool may be configured for holding and precise placement of cochlear implants during surgery. The inventive system allows for the safe and precise placement of cochlear implants in patients in a context where performing this procedure manually without the inventive device is beyond the limits of human dexterity. The insertion tool for placement of cochlear implants may comprise a non-sterile handpiece incorporating electrical and mechanical components, an element for coupling the handpiece to a fine adjustment apparatus, a sterile adapter and a clip portion for holding a cochlear implant. Provided is thus an inventive system that ensures quality cochlear implant insertion through safe and precise placement of cochlear implants in a manner that goes beyond the limits of ordinary human dexterity.

## Description

### FIELD OF THE INVENTION

The invention relates to a system for moving objects in a sterile environment by conveying motion created in a nonsterile environment and conveyed through a sterile barrier and towards the sterile environment. In certain embodiments, the system may include a sterilizable member fitted with an adapter for holding a tool that fits over a non-sterilizable member, wherein movement of a magnetic source inside the non-sterilizable member conveys motion to the adapter acting as a ferromagnetic sink. In particular, the adapter may be fitted with a tool that may function to move an object requiring precise placement or manipulation. In various embodiments, the present invention relates to objects (tools or implants) that must be moved during a surgical procedure. In particular, the present invention pertains to movement of objects in contexts where movements with precise speed and acceleration are required. In certain embodiments, the movement of objects of the present invention may be in a precise and predefined spatial position and/ or orientation for which a fine adjustment apparatus which may also optionally be coupled with a surgical positioning arm that may or may not be a robotic arm may be required. More particularly, the instant invention relates to the movement of surgical tools for use in procedures where precision in the spatial and temporal aspects of the movement is required for success. Most particularly, the present invention pertains to surgical instruments for use in placement of cochlear implants during cochlear implant surgery. The insertion tools of the present invention may optionally be used with a fine adjustment mechanism for even greater control of insertion and may also be coupled with a surgical positioning arm that may optionally be a robotic arm. In addition, various embodiments of the present invention relate to surgical insertion tools with sterilizable or disposable components and non-disposable components joined by a coupling means that may be magnetic.

### BACKGROUND OF THE INVENTION

In all surgical applications, precise movement or placement of sterile devices in a sterile field is required. This requirement can also find application in various clinical and or industrial fields, such as the manipulation of tissue, in precision manufacturing, and also in the medical and/or surgical field. In each application, a cost-effective approach may be required for precise movement and/or placement of devices or tools.

One problem that may be encountered in each of these applications is that the machinery or apparatus required for precise consistent axial movement (i.e. minimal variations of speed of movement) may be rather complex in its technical implementation and therefore not cost effective to be disposed after each cycle of use. Hence, non-disposable, but also non-sterilizable implementations are required. Accordingly, one solution approach may be to fit a non-sterilizable component containing a precise and expensive movement actuator with a component that is rather non-complex, but acts as a "transmission" component and is either robust and therefore re-processable and or even simple and therefore cost effective to be disposable after each use. In this approach, a solution for conveying precise motion between the non-sterilizable and sterilizable components may be required. Applicants are unaware of a solution of this sort being currently available and they provide a cost-effective and precise solution herein.

While a solution for conveying precise motion from a non-sterilizable component of an apparatus to a sterilizable component is broadly applicable across scientific, manufacturing and medical fields, specific applications in the surgical field are apparent. Many surgical applications require precise manipulation of surgical tools and/or precise placement of medical devices. This provides a clear example of an instance where high precision is required and, thus, the machinery providing the precise motion is likely expensive and may be non-sterilizable. Accordingly, conveying the precise motion from the non-sterilizable portion to a sterilizable portion would be highly desirable. Otological surgery is a key example - the structures being operated on are very small and there is low margin for error. Cochlear implant surgery is one example of an otological surgery procedure that may benefit from such an approach.

Cochlear implant surgery ("CI") consists of three main steps. First, a mastoidectomy is performed, whereby the mastoid bone is opened, providing access to the middle ear space. Next, the surgeon navigates the middle ear space, taking care to avoid key structures such as the facial nerve and chorda tympani. Once entered into the middle ear cavity, the surgeon creates an opening either into the round window membrane of the cochlea (round window approach) or into the cochlea wall (cochleostomy). Finally, the electrode array is inserted into the cochlea. Each step of CI carries risks and challenges, including the possibility of damage to nerves lying in the lateral skull base, incorrect or incomplete insertion of the electrode array, damaging of intracochlear structures or sub-optimal choice of cochlear implant for the individual patient.

Computer assisted planning of CI procedures has been described utilizing medical images, to identify relevant anatomy (e.g., Cochlear) to plan for access routes into the cochlea. To date commercial tools such as OTOPLAN are used to assist in an optimal electrode choice with respect to available imaging and audiological data of a given patient. (selecting the desired length of the CI electrode with respect to anatomy).

Various aspects and steps of a cochlear implantation procedure involve risk to the patient, including the drilling, proper electrode selection and, critically, the step of actually inserting the chosen electrode into the cochlea. Robotic cochlear implant insertion (RCI) has not achieved anywhere near universal adoption due to factors such as cost, relative infancy of the underlying technology, and lack of a quality approach to robotic insertion. Accordingly, current practice still involves manual surgical insertion of the implant electrode that is inherently destructive. Inserting a cochlear implant is actually beyond the limits of human dexterity and so approximately 50% of cochlear implant patients do not have the hearing performance they need after surgery. This is generally because the insertion process is so rough and destructive with respect to the inner ear structures, which can often result in destruction of any residual hearing the patient has. Accordingly, ordinary CI surgery is only cleared for deaf patients. Because of this dynamic, along with cost issues, implementation rates of cochlear implants in general are as low as 5%. As a result, in a worldwide population of 15 million with hearing problems that may be addressed by cochlear implants, perhaps only 70,000 actually receive implants

The goal of quality cochlear implant insertion has not yet been achieved in the field, where quality insertion can be defined as the ability to place cochlear implants safely and accurately - without damage to surrounding structures while achieving a placement that truly enhances the patient's hearing to a worthwhile extent. Quality cochlear implantation involves planning the surgical approach and patient specific implant selection (using dedicated software based decision support systems, such as OTOPLAN), monitoring of insertion progress (using either fluoroscopy or electro-cochleography - impedance measurements across the different electrodes) and, critically, an actual insertion process that minimizes variations of insertion speed to avoid damage to the delicate structures of the inner ear.

It has been claimed, that cochlear implantation is literally beyond human dexterity and thus there is a strong need in the clinical field for an insertion tool and methodology that can provide for precise, safe insertion of implants through insertions with the greatest level of consistency, ie minimum variations of insertion speed.

Manual insertion of cochlear implants involves use of surgical tools like forceps or stylets. However, these surgical tools are currently moved manually, and, as discussed herein, it is understood that achieving consistency in movements is certainly beyond the limits of human mechanical dexterity, hence damage to the cochlear structures must be expected. Also, as is well known to surgeons and others of skill in the art, insertion of a cochlear implant in addition is challenging due to the limited space that the surgical approach (mastoidectomy, posterior tympanotomy) provides and as a result access for manually operated surgical instruments is extremely limited.

The difficulty of electrode array insertion is often compounded by the existing anatomical variations of the involved structures (size of cochlea, position and orientation of round window) between patients, meaning that different challenges are faced in each surgical procedure. For example, the round window opening of the cochlea is not always well exposed through the facial recess in all patients, thus forcing the surgeon to navigate without good visibility in a limited space to place a small electrode array with imperfect instruments.

Thus, there is a strongly felt need in the field for an insertion tool and system that provide for precise control of placement of cochlear implants during CI procedures. Also required is an insertion tool and system that provides disposable or sterilizable relatively inexpensive insertion components that are designed for use in the sterile field that can be coupled with non-disposable, relatively expensive mechanical and electrical components that ultimately provide the needed precision of movement of the disposable/sterilizable components.

Various strategies and tools have been suggested for improving precision and safety of cochlear implant insertion. For example, the present assignees in WO2022/024090 propose an approach involving surgical planning, electrode selection and monitoring insertion progress. In US8473075 Advanced Bionics discloses an insertion system that includes a stiffening element that provides for insertion of the electrode array without buckling. In US7894916 Cochlear discloses an insertion apparatus involving shape memory material that helps adapt the electrode array to the shape of the patient's cochlea. And in US10994128, Med El discloses an insertion apparatus that includes a retraction limiter and a plunger coupled with the implant to help ensure appropriate placement of the electrode array.

However, none of these known approaches and apparatuses provide an insertion tool that can be coupled with a stage that can provide effective movement of the tool in selected spatial degrees of freedom. Such a stage can optionally take the form of a fine adjustment apparatus and, optionally, a surgical arm that accordingly allow for insertion of a cochlear implant with spatial and temporal precision that goes beyond that of human dexterity. In this regard, it is understood that known approaches may improve implant placement, but that only a system like the present invention that literally takes effective movement of the implant during the insertion process out of the surgeon's hands can provide the needed precision to maximally reduce structural damage to the cochlear.

Keeping these drawbacks in mind, the current inventors have realized that a multifaceted insertion tool involving a disposable/sterilizable component, coupled with non-disposable elements that may be configured to hold and precisely place cochlear implants during surgery is needed for quality, safe CI procedures.

Those of skill in the art will realise of course that the same need is felt in any surgical field requiring precise movement control and/or precise placement of medical devices. More broadly, scientific or manufacturing fields requiring precise movement control but also cost-effective use of sterile components will also benefit.

### SUMMARY OF THE INVENTION

These aims and other advantages are achieved by a new system for conveying motion from a non-sterilizable component to a sterilizable component. In various embodiments, motion is conveyed from a ferromagnetic source inside a sterilizable component to a ferromagnetic sink fitted to a sterilizable component. In various specific embodiments, the ferromagnetic sink may function as an adapter that can be further fitted with a tool that can be advanced in a sterile field and/or manipulate objects in a sterile field.

In some embodiments, the system is configured such that the sterilizable member can be rigidly attached and detached reproducibly over the non-sterilizable member and such that the motion of the moving element of the non-sterilizable member is conveyed to the moving element of the sterilizable member through magnetic coupling of the ferromagnetic source with the ferromagnetic sink.

In further embodiments, the magnetic coupling between the magnetic source and the magnetic sink may be selectively engaged and disengaged. Selective disengagement can occur at a predetermined load and may be carried out for safety reasons in the particular application. Selective engagement or disengagement may also be controlled through modulation of an electrical current running through the non-sterilizable component in proximity to the ferromagnetic source.

In some embodiments, these aims and other advantages are also achieved by a new system for the quality insertion of cochlear implants by surgical means that may include open, minimally invasive or robotic. The inventive system is an insertion tool that may comprise a hand piece incorporating electrical and mechanical components, a sterile adapter, an electrode clip, a piezo-motorbox and a foot pedal. Those of skill in the art will understand that not all of these components will be required for an insertion tool that functions for precise placement of cochlear implants or other medical devices. Accordingly, elements may be omitted or modified and the clip may be configured to hold and place a medical device other than an electrode array. The insertion device is nonetheless optimally configured for the precise placement of a cochlear implant or other medical device requiring precise placement.

Those of skill in the art will thus understand that the inventive insertion device may also be used for insertion of other medical devices besides just cochlear implants. Any surgical context where a medical device has to be inserted with great care given to precision and placement will be amenable to use of the current insertion device. Examples given herein relating to the insertion of cochlear implants are representative in nature but are not exclusive.

Thus, according to an embodiment of the current invention, a system for precise and safe placement of a medical device is provided. The inventive system may comprise a non-sterile handpiece with proximal and distal ends, a coupling element for coupling the portion of the handpiece adjacent to its distal end to an apparatus for fine adjustment of the position of the system, a sterile adapter portion detachably affixed to the distal end of the handpiece comprising a tube with proximal and distal ends and a rotation sleeve engaged to the proximal end of the tube, wherein the rotation sleeve is detachably affixable to the distal end of the handpiece, and a clip portion slidably engaged around the tube of the sterile adapter portion. The system is optimally configured to advance a medical device held by the clip into position in a patient.

In an embodiment, the system is an insertion tool for a cochlear implant, the medical device is a cochlear implant and the patient is a patient suffering from hearing loss amenable to treatment with an appropriately selected and placed cochlear implant.

In an embodiment, an inner circumferential portion of the rotation sleeve of the sterile adapter portion comprises teeth that are engageable with corresponding teeth on an outer circumferential portion of the distal end of the handpiece so as to provide a secure, detachable fixing of the sterile adapter to the handpiece.

In a further embodiment, the tube of the sterile adapter portion comprises a magnet slidably disposed in an inner lumen of the tube and wherein the clip portion is constructed of a magnetic material such that the position of the clip portion on the tube can be controlled by engagement with the magnet.

In a further embodiment, the rotational position of the clip portion can be adjusted by selectively engaging and rotating the teeth of the rotation sleeve around the teeth of the distal end of the handpiece.

In a further embodiment, the apparatus for fine adjustment of the position of the system comprises an attachment element for selective engagement to the coupling element and wherein the apparatus provides for adjustment in at least 3 vectors of the position of the medical device held by the clip.

The inventive system of the present invention may optimally be used in a surgical approach to cochlear implantation, where the surgical approach may be open, minimally invasive or robotic. All surgical approaches to cochlear implantation will benefit from the precision in cochlear implant placement provided by the current insertion tool once access to the patient's cochlea has been established. All surgical approaches require an insertion system that can precisely place a cochlear implant without damaging the inner ear structures, which is understood to be beyond the limits of human dexterity. Therefore, an insertion tool that comprises a piezo-motorized component along with a fine adjustment modality, such as that of the present invention, will be beneficial, and indeed required, for reliably injury-free cochlear implant placement and positioning.

Optionally, the insertion tool of the present invention may be combined with surgical planning and anatomically correct electrode placement and intra-operative monitoring to reach the goal of quality cochlear implant placement. Even deployed on its own, however, the inventive insertion tool will enhance quality in CI procedures because it will provide for implant placement and positioning with a precision that goes beyond human dexterity, thus reducing injury and improving surgical results. While this level of precision may eventually be able to be provided by adoption of RCI procedures, in the context where RCI adoption is low, an insertion tool that provides for precise implant placement in minimally invasive or open procedures is critical.

These and other embodiments of the inventive system and method are described in more detail below with reference to the attached figures.

### BRIEF DESCRIPTION OF THE FIGURES

- Figures 1, 1a and 1b show a side cross-sectional view of a system for conveying motion from a non-sterilizable portion to a sterilizable portion according to various embodiments of the present invention.
- Figures 2, 2a and 2b show a perspective cut-away view of a system for conveying motion in two dimensions from a non-sterilizable portion to a sterilizable portion according to various embodiments of the present invention.
- Figure 3 is a side view of an insertion device comprising sterile and non-sterile components according to an embodiment of the present invention.
- Figure 4 provides close-up side views of an electrode clip and its components according to an embodiment of an insertion tool of the present invention.
- Figure 5 provides close-up views of a sterile adapter and its components according to an embodiment of an insertion tool of the present invention.
- Figure 6 shows close-up side views of a hand piece and its components according to an embodiment of an insertion tool of the present invention.
- Figure 7 demonstrates a component for coupling a hand piece to a fine adjustment apparatus according to an embodiment of an insertion tool of the present invention.
- Figure 8 shows a fine adjustment apparatus for coupling to a handpiece according to an embodiment of an insertion tool of the present invention.
- Figure 9 shows an elevated side view of a representative surgical setup for use of an insertion tool according to an embodiment of the present invention.

### DETAILED DESCRIPTION OF THE INVENTION

The present invention is now described in detail in connection with its various embodiments and with reference to the attached figures.

In an embodiment, a system for conveying motion from a non-sterilizable component to a sterilizable component is provided. The system may comprise a non-sterilizable member comprising a moving element containing a passive or active ferromagnetic source, a sterilizable member comprising a moving element containing a passive ferromagnetic sink; and an adapter attached to the moving element of the sterilizable member to which objects such as surgical instruments, implants or the like can be attached temporarily. The system may be configured such that the sterilizable member can be rigidly attached and detached reproducibly over the non-sterilizable member and such that the motion of the moving element of the non-sterilizable member is conveyed to the moving element of the sterilizable member through magnetic coupling of the ferromagnetic source with the ferromagnetic sink.

In some embodiments, the conveyed motion causes motion of the moving element of the sterilizable member and consequently causes motion of the attached object in a sterile field. The motion may be translational or rotational.

In some embodiments, the magnetic coupling between the ferromagnetic source and the ferromagnetic sink can be selectively engaged and disengaged. The selective disengagement can occur when a predetermined load is reached, for example, when motion in a sterile field is not desirable.

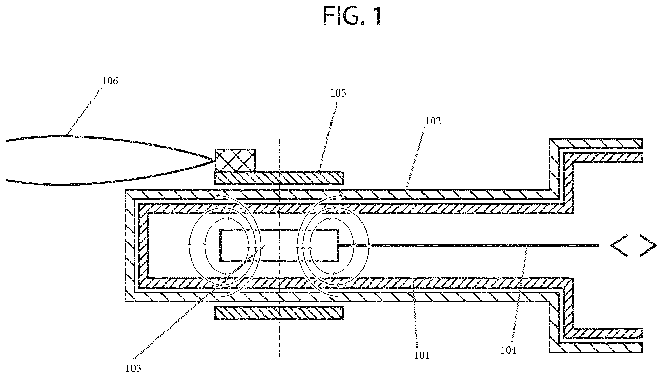

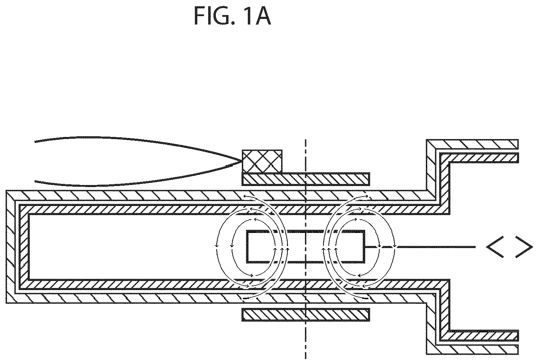

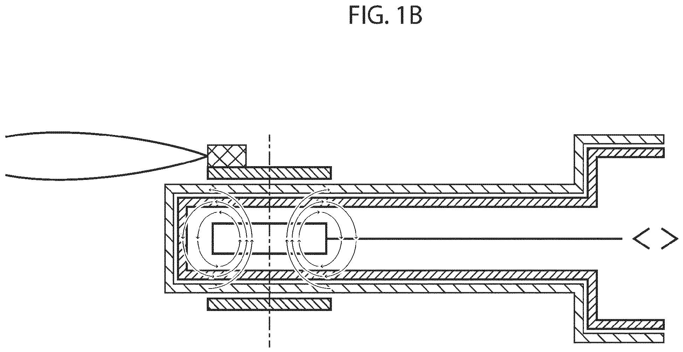

With reference now to Figure 1, a system for conveying motion may comprise a non-sterilizable component 101 and a sterilizable component 102. A ferromagnetic source 103 may be placed inside the non-sterilizable component 101. An actuator 104 may be provided that may function to move the ferromagnetic source 103. A ferromagnetic sink 105 may be fitted to the sterilizable portion 102. The ferromagnetic sink 105 may function as an adapter for connection to a tool 106. In the embodiment of Figure 1, movement of the actuator 104 causes movement of the ferromagnetic source 103. Consequently, movement of the ferromagnetic sink 105 or adapter also occurs, further consequently causing motion of the tool 106. In the embodiment of Figure 1, the sterilizable component, adapter and tool may be sterilized and therefore deployed in a sterile field, while the non-sterilizable component may provide precise control of motion without need for sterilization.

Figures 1a and 1b show the components of the embodiment of the inventive system in different positions based upon movement of the actuator forward and backward in a horizontal direction.

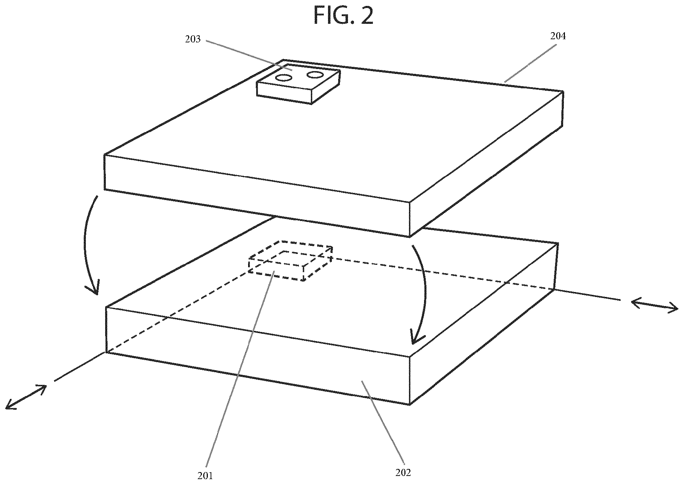

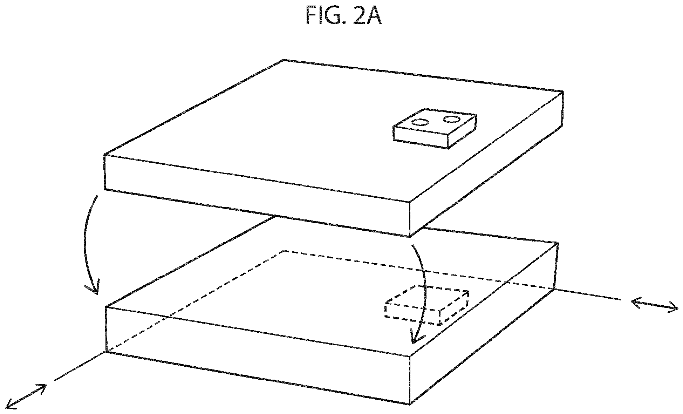

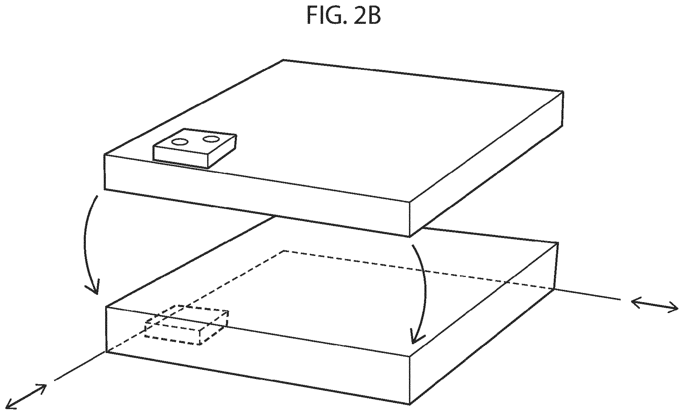

With reference now to Figure 2, a ferromagnetic source 201 may be placed inside a non-sterilizable component 202 and may be used to convey motion in two dimensions to a ferromagnetic sink 203 attached to a sterilizable component 204. As in the embodiment of Figure 1, the ferromagnetic sink may function as an adapter for further coupling to a tool (not shown). Accordingly, precise movement of a tool in two dimensions in a sterile field can be provided.

Figures 2a and 2b show the components of the embodiment of the inventive system in different positions based upon movement of the ferromagnetic source along two dimensions of motion.

In a further specific embodiment, a system for precise and safe placement of a medical device is provided. The inventive system is an insertion tool that may comprise a hand piece incorporating electrical and mechanical components, a sterile adapter, an electrode clip, a piezo-motorbox and a foot pedal. The inventive system may comprise a non-sterile handpiece with proximal and distal ends, a coupling element for coupling the portion of the handpiece adjacent to its distal end to an apparatus for fine adjustment of the position of the system, a sterile adapter portion detachably affixed to the distal end of the handpiece comprising a tube with proximal and distal ends and a rotation sleeve engaged to the proximal end of the tube, wherein the rotation sleeve is detachably affixable to the distal end of the handpiece, and a clip portion slidably engaged around the tube of the sterile adapter portion. The system is optimally configured to advance a medical device held by the clip into position in a patient. The medical device may be a cochlear implant and the clip may be a pair of forceps or other suitable surgical tool for holding a cochlear implant.

The piezo-motorbox may be incorporated into or coupled to the handpiece to allow for advancement and withdrawal of the clip or forceps forward or backward into the surgical field. For example, the piezo-motorbox may be incorporated into or coupled to the handpiece of an insertion tool for the insertion of cochlear implants during CI procedures. It may thus provide for the controlled advancement into a patient's inner ear of a pair of forceps holding a cochlear implant or for the withdrawal of the forceps holding a cochlear implant. One of skill in the art will understand that elements other than a piezo-motorbox may provide this advancement and/or withdrawal function, but that an element providing for controlled, precise advancement and retraction is required to meet the goal of safe and precise placement of the cochlear implant. In various embodiments, a foot pedal may allow for operator control of the piezo-motorbox but those of skill in the art will understand that any similarly functioning element usable in an operating room that can start and stop advancement or withdrawal of the clip or forceps will be acceptable.

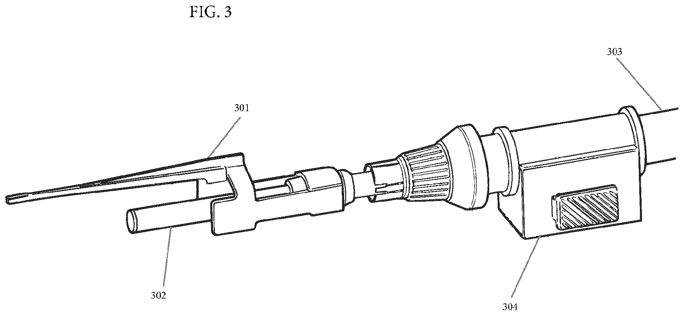

With reference now to Figure 3, a side view of an insertion device comprising sterile and non-sterile components according to an embodiment of the present invention is shown. The insertion device comprises a clip element 301 that may be configured to hold an electrode array of a cochlear implant or any other medical device that requires precise handling and/or insertion. The clip element 301 may be in the form of a pair of forceps or any other suitable holding device. The clip element 301 may be formed from one or more interlocking sliding sleeve elements that fit together while also circumferentially fitting around a sterile adapter 302. The sterile adapter 302 and the clip element 301 are designed to be disposable or sterilizable and can thus be deployed in the sterile field during surgery. The sterile adapter 302 is configured to be attachable to a hand piece 303 that can be held and manipulated by a surgeon during a surgical procedure. The handpiece 303 is not necessarily sterile and is not necessarily designed to be disposable or reprocessable. The handpiece 303 may contain electrical or mechanical elements for operation of the insertion device.

Still with reference to Figure 3, the handpiece 303 may also be coupled to a coupling element 304 that may be configured to couple the handpiece to a piezo-motorbox (not shown) or other apparatus for controlling movement of the insertion device. One of skill in the art will understand that all elements of the insertion device may be constructed of materials known to be suitable for construction and operation of similar devices and also suitable for use in an operating room setting.

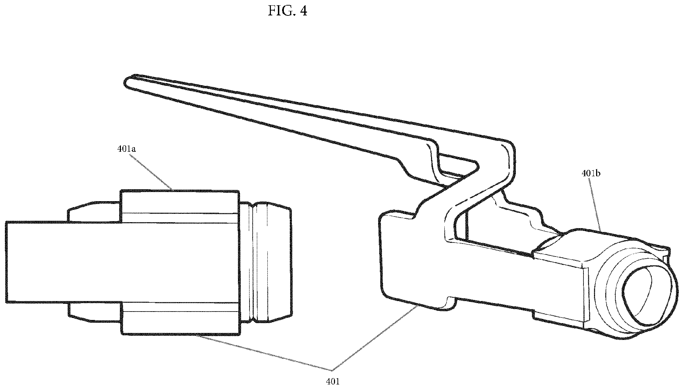

With reference now to Figure 4, a close-up side views of an electrode clip and its components according to an embodiment of an insertion tool of the present invention is provided. As disclosed with reference to Figure 3, the clip element 301 may be formed from one or more interlocking sliding sleeve elements that fit together while also circumferentially fitting around a sterile adapter 302. In Figure 4, a close-up view of these elements of a clip element 401 are shown. Specifically, two interlocking sliding sleeve elements 401a and 401b are shown. These elements are configured to interlock together and to circumferentially fit around a sterile adapter portion of a insertion device. The sliding sleeve elements 401a and 401b are exemplary in nature. Many variations are possible with the basic requirement being that the elements may interlock together, may form together a surgical tool suitable for holding or advancing a medical device in a surgical field, and may fit circumferentially around a sterile adapter or other similar component of an insertion tool according to the teachings of the current invention. The sliding sleeve elements 401a and 401b may have a circumferential groove to allow for proper manual fixation to the sterile adapter portion.

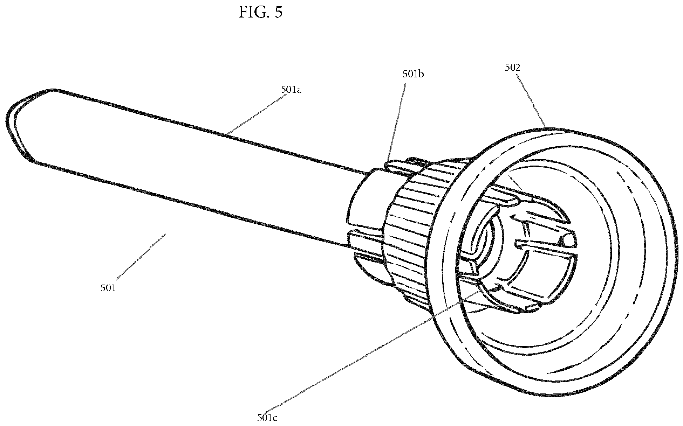

With reference now to Figure 5, a close-up views of a sterile adapter 501 and its components according to an embodiment of an insertion tool of the present invention is provided. The sterile adapter may be composed of three or more key elements. First, the portion 501a may be a sterile tube in a polygon shape. Second, a rotation sleeve 502 may fit around the sterile tube 501a. The rotation sleeve 502 may present snap-in springs 501b or other suitable elements for fixation to the clip element. The rotation sleeve 502 may also present additional snap-in springs 501c on the inner edge (or other suitable elements) for fixation to the handpiece. With this design, the sterile adapter provides for reliable fixation to the clip element and to the handpiece, ultimately providing for a stable, sterile connection between the handpiece and the clip element.

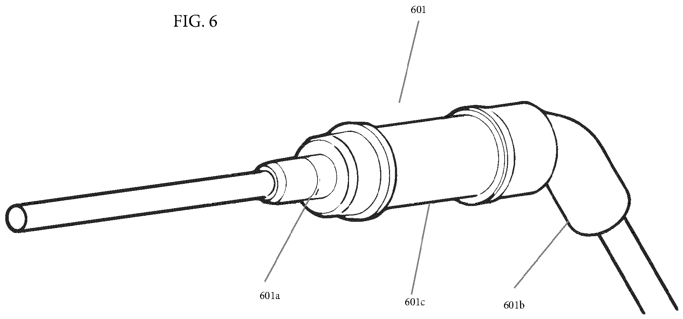

With reference now to Figure 6, a close-up side views of a hand piece and its components according to an embodiment of an insertion tool of the present invention is provided. The handpiece 601 provides a circumferential groove 601a for fixation of the rotation sleeve of the sterile adapter. The body of the handpiece 601b is optimally rotationally symmetrical and constructed of stainless steel. The handpiece 601 also may provide a snap-in groove 601c for attachment to a mounting component that may serve to fix the handpiece to a piezo-motorbox or other motorized element for movement and/or advancement of the insertion tool in the surgical field.

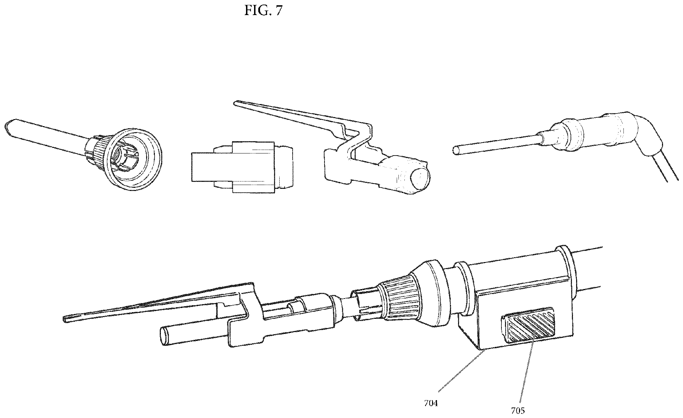

With reference now to Figure 7, a component for coupling a hand piece to a fine adjustment apparatus according to an embodiment of an insertion tool of the present invention is demonstrated. The coupling element 704 may incorporate a spring loaded snap coupling. This spring loaded snap coupling may be configured, with reference back to Figure 6, to a snap-in groove 601c of a handpiece 601. The coupling element 704 may be released from a handpiece of an insertion tool with release buttons 705. The coupling element 704 may also incorporate means for fixation onto a fine adjustment mechanism via screws, not shown.

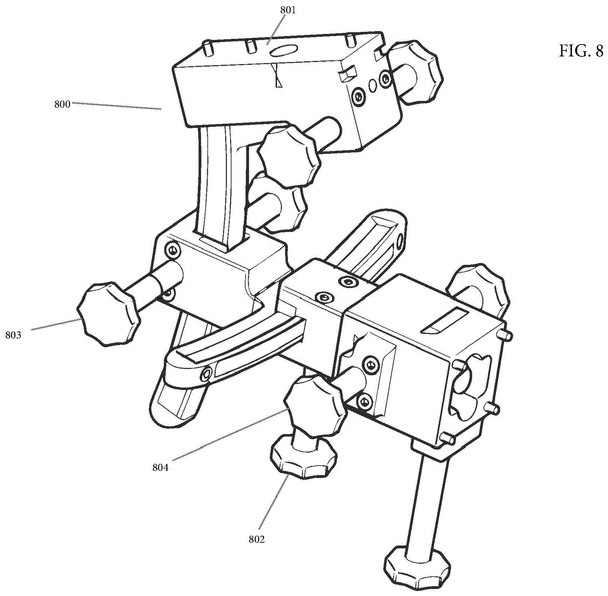

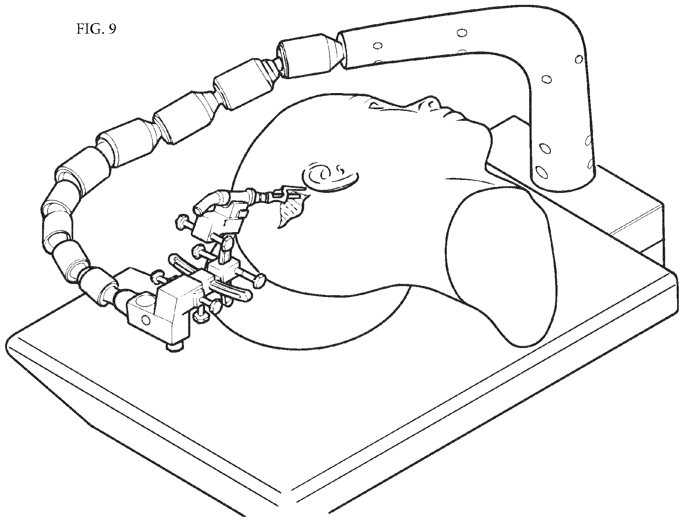

With reference now to Figure 8, a fine adjustment apparatus 800 for coupling to a handpiece according to an embodiment of an insertion tool of the present invention is shown. The fine adjustment mechanism incorporates an interface coupling 801 that allows for the fine adjustment mechanism to be coupled a handpiece through a coupling element such as that shown in Figure 7. With specific reference back to Figure 7, the coupling element 704 comprises means for fixation to a fine adjustment mechanism such as that shown in Figure 9 through screws or other suitable fixation elements. The fine adjustment apparatus of Figure 8 also may comprise a linear z adjustment 802, a vertical horizontal adjustment 803 and a linear x/y adjustment 804. All of the elements 802, 803 and 804 allow for very precise and fine adjustment of movement of the fine adjustment apparatus that in turn allow for very precise, fine and controllable movement of, for example, an insertion tool that the fine adjustment apparatus may be coupled with. The fine adjustment apparatus also may comprise a further coupling mechanism 805 that may serve to couple the fine adjustment apparatus to a device such as, solely by way of example, a manual or robotic surgical arm.

One of skill in the art will now understand that the invention disclosed herein is an insertion tool for use in surgical settings where precise control of insertion of a medical device is required. In the various embodiments presented, the handpiece of the insertion tool is coupled to, or integrated with, motorized and/or mechanical and/or electrical components that allow for controlled movement of a clip portion or other holder for a medical device, such as an electrode array of a cochlear implant. A fine adjustment mechanism can also be integrated, providing for even finer control of movement in surgical applications where such control is required to achieve desired results.

The insertion device also addresses key needs by having sterile and non-sterile components. In exemplary embodiments, the insertion device has a clip element that may be disposable or sterilizable and that is configured to fit around a sterile adapter portion that can also be disposable or sterilizable. Meanwhile, the sterile adapter portion can be fitted to a handpiece that contains or is coupled to all of the apparatus portions that contain electrical or mechanical or motorized components. The handpiece and other elements that it is coupled to that contain electrical or mechanical or motorized components are not configured to be disposable or sterilizable. Thus, the more expensive elements of the insertion device are kept outside the sterile field and can be constructed of appropriate materials and do not need to be reprocessed, meaning that they can have a much longer lifetime before requiring replacement. This strategy keeps overall cost of the insertion device and its components to a minimum.

With reference now to Figure 9, a representative surgical setup is shown for use of the inventive insertion tool. In this exemplary embodiment, the surgical setup is prepared for insertion of a cochlear implant to the inner ear of a patient. The insertion tool is shown coupled to a fine adjustment apparatus which, in turn, is further coupled to a surgical arm. The demonstrated surgical setup provides for precise control of movement of the insertion tool during the surgery and, thus, precise and controllable placement of an electrode array of a cochlear implant in the patient.

While this invention has been shown and described with reference to particular embodiments thereof, it will be understood by those of skill in the art that various changes in form and details may be made therein without departing from the spirit and the scope of the invention as defined by the appended claims. Solely by way of example, not by way of limitation, one of skill in the art will easily understand that the methods/systems disclosed herein can be applied to other surgical fields.

In the following further aspects of the present inventions and embodiments thereof are listed as items. These items may however also be formulated as claims of the present application:
Item 1: A system for moving objects by conveying motion through a sterile barrier, comprising:
   - a non-sterilizable member comprising a moving element containing a passive or active ferromagnetic source;
   - a sterilizable member comprising a moving element containing a passive ferromagnetic sink; and
   - an adapter attached to the moving element of the sterilizable member to which objects such as surgical instruments, implants or the like can be attached temporarily.
   wherein the system is configured such that the sterilizable member can be rigidly attached and detached reproducibly over the non-sterilizable member and such that the motion of the moving element of the non-sterilizable member is conveyed to the moving element of the sterilizable member through magnetic coupling of the ferromagnetic source with the ferromagnetic sink.
Item 2: The system of item 1, wherein the conveyed motion causes motion of the moving element of the sterilizable member and consequently causes motion of the attached object in a sterile field.
Item 3: The system of item 2, wherein the attached object's effective motion is translational or rotational or both translational and rotational.
Item 4: The system of any of the previous items wherein the system allows creation of motion in more than one degree of freedom.
Item 5: The system of item 4, wherein the effective motion of the object is enabled while all other degrees of motion are blocked.
Item 6: The system of item 1 wherein the source of ferromagnetic field is static.
Item 7: The system of item 6, wherein the source of ferromagnetic field is a permanent magnet.
Item 8: The system of item 1, wherein the source of ferromagnetic field is dynamic.
Item 9: The system of item 8, wherein the source of ferromagnetic field is dynamically controlled.
Item 10: The system of item 1, wherein the magnetic coupling between the ferromagnetic source and the ferromagnetic sink can be selectively engaged and disengaged.
Item 11: The system of item 10, wherein the magnetic coupling is selectively disengaged at a predetermined load.
Item 12: The system of item 11, wherein the magnetic coupling is selectively disengaged when motion of the sterilizable member in the sterile field is not desirable.
Item 13: The system of item 12, wherein motion is not desirable because the moving object attached to the actuator encounters an obstacle or because motion is restricted due to increased physical resistance.
Item 14: The system of any of items 10 through 13, wherein the selective disconnection of the interaction is controlled by the modulation of the field strength of the ferromagnetic source.
Item 15: The system of item 14, wherein the modulation of the source of the ferromagnetic field is accomplished through dynamic control of an electric current running through a coil in the non-sterile member.
Item 16: The system of item 15 wherein the adapter is configured to position/place an implantable medical device in a patient, wherein the implantable medical device is selectively grasped by a forceps or clamp or other grasping instrument.
Item 17: The system of item 16 wherein the implantable medical device is a cochlear implant.
Item 18: The system of any of items 1 through 17 wherein the sterilizable member is configured to position an injector in a patient.
Item 19: The system of any of the preceding items wherein the sterilizable member is a sleeve that fits over a sterile barrier and the non-sterilizable member.
Item 20: The system of any of the preceding items wherein the sterilizable member is configured for single-use only.
Item 21: The system of any of the preceding items wherein the sterilizable member is configured for multiple-use.
Item 22: A system for precise and safe placement of a medical device comprising
   a non-sterile handpiece with proximal and distal ends;
   a coupling element for coupling the portion of the handpiece adjacent to its distal end to an apparatus for fine adjustment of the position of the system;
   a sterile adapter portion detachably affixed to the distal end of the handpiece comprising
      a tube with proximal and distal ends; and
      a rotation sleeve engaged to the proximal end of the tube, wherein the rotation sleeve is detachably affixable to the distal end of the handpiece; and
   a clip portion slidably engaged around the tube of the sterile adapter portion;
   wherein the system is configured to advance a medical device held by the clip into position in a patient.
Item 23: The system of item 22, wherein an inner circumferential portion of the rotation sleeve comprises teeth that are engageable with corresponding teeth on an outer circumferential portion of the distal end of the handpiece so as to provide a secure, detachable fixing of the sterile adapter to the handpiece.
Item 24: The system of item 23, wherein the tube comprises a magnet slidably disposed in an inner lumen of the tube and wherein the clip portion is constructed of a magnetic material such that the position of the clip portion on the tube can be controlled by engagement with the magnet.
Item 25: The system of item 23, wherein the rotational position of the clip can be adjusted by selectively engaging and rotating the teeth of the rotation sleeve around the teeth of the distal end of the handpiece.
Item 26: The system of item 22, wherein the apparatus for fine adjustment of the position of the system comprises an attachment element for selective engagement to the coupling element and wherein the apparatus provides for adjustment in at least 3 vectors of the position of the medical device held by the clip.

## Claims

1. A system for moving objects by conveying motion through a sterile barrier, comprising:
- a non-sterilizable member comprising a moving element containing a passive or active ferromagnetic source;
- a sterilizable member comprising a moving element containing a passive ferromagnetic sink; and
- an adapter attached to the moving element of the sterilizable member to which objects such as surgical instruments, implants or the like can be attached temporarily.
wherein the system is configured such that the sterilizable member can be rigidly attached and detached reproducibly over the non-sterilizable member and such that the motion of the moving element of the non-sterilizable member is conveyed to the moving element of the sterilizable member through magnetic coupling of the ferromagnetic source with the ferromagnetic sink.

2. The system of claim 1, wherein the conveyed motion causes motion of the moving element of the sterilizable member and consequently causes motion of the attached object in a sterile field.

3. The system of claim 2, wherein the attached object's effective motion is translational or rotational or both translational and rotational.

4. The system of any of the previous claims wherein the system allows creation of motion in more than one degree of freedom.

5. The system of claim 4, wherein the effective motion of the object is enabled while all other degrees of motion are blocked.

6. The system of claim 1 wherein the source of ferromagnetic field is static.

7. The system of claim 6, wherein the source of ferromagnetic field is a permanent magnet.

8. The system of claim 1, wherein the source of ferromagnetic field is dynamic.

9. The system of claim 8, wherein the source of ferromagnetic field is dynamically controlled.

10. The system of claim 1, wherein the magnetic coupling between the ferromagnetic source and the ferromagnetic sink can be selectively engaged and disengaged.

11. The system of claim 10, wherein the magnetic coupling is selectively disengaged at a predetermined load, wherein particularly the magnetic coupling is selectively disengaged when motion of the sterilizable member in the sterile field is not desirable.

12. The system of claim 11, wherein motion is not desirable because the moving object attached to the actuator encounters an obstacle or because motion is restricted due to increased physical resistance.

13. The system of any of claims 10 through 12, wherein the selective disconnection of the interaction is controlled by the modulation of the field strength of the ferromagnetic source, wherein particularly the modulation of the source of the ferromagnetic field is accomplished through dynamic control of an electric current running through a coil in the non-sterile member, and wherein particularly the adapter is configured to position/place an implantable medical device in a patient, wherein the implantable medical device is selectively grasped by a forceps or clamp or other grasping instrument.

14. The system of claim 13 wherein the implantable medical device is a cochlear implant.

15. The system of any of the preceding claims wherein the sterilizable member is a sleeve that fits over a sterile barrier and the non-sterilizable member.
